(19) Europäisches Patentamt
European Patent Office
Office européen des brevets

(11) EP 2 344 036 B1

(12) **EUROPÄISCHE PATENTSCHRIFT**

(45) Veröffentlichungstag und Bekanntmachung des Hinweises auf die Patenterteilung:
**06.06.2012 Patentblatt 2012/23**

(21) Anmeldenummer: 09740638.3

(22) Anmeldetag: **10.09.2009**

(51) Int Cl.:
*A61B 5/091* (2006.01)     *G01F 1/66* (2006.01)
*G01N 29/07* (2006.01)

(86) Internationale Anmeldenummer:
**PCT/DE2009/001284**

(87) Internationale Veröffentlichungsnummer:
**WO 2010/040332 (15.04.2010 Gazette 2010/15)**

(54) **VERFAHREN ZUR LUNGENTOTRAUMMESSUNG**

METHOD FOR MEASURING DEAD LUNG SPACE

PROCÉDÉ DE MESURE DE L'ESPACE MORT PULMONAIRE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **07.10.2008 DE 102008050497**

(43) Veröffentlichungstag der Anmeldung:
**20.07.2011 Patentblatt 2011/29**

(73) Patentinhaber: **Ganshorn Medizin Electronic GmbH**
**97618 Niederlauer (DE)**

(72) Erfinder: **GANSHORN, Peter**
**97702 Münderstadt (DE)**

(74) Vertreter: **Pöhner, Wilfried Anton**
**Patentanwalt,**
**Röntgenring 4**
**97070 Würzburg (DE)**

(56) Entgegenhaltungen:
**EP-A2- 0 145 384        DE-A1-102004 039 194**
**US-A1- 2001 049 478**

• BHAVANI-SHANKAR K ET AL: "Defining segments and phases of a time capnogram." ANESTHESIA AND ANALGESIA OCT 2000, Bd. 91, Nr. 4, Oktober 2000 (2000-10), Seiten 973-977, XP002562634 ISSN: 0003-2999
• BUESS CH ET AL: "ULTRASONIC RESPIRATION ANALYSIS" PROCEEDINGS OF THE ANNUAL INTERNATIONAL CONFERENCE OF THE IEEE ENGINEERING IN MEDICINE AND BIOLOGY SOCIETY, Bd. 13, Nr. 4, 31. Oktober 1991 (1991-10-31), Seiten 1597-1598, XP000348108

**Beschreibung**

Die Erfindung bezieht sich auf ein Verfahren zur Bestimmung des anatomischen Totraumes im Atemtrakt von Lebewesen durch die kontinuierliche und simultane Messung des Flow (F) und der Atemluftdichte(D) beim Ausatmen (EX) über die Zeit (T) hinweg.

Zum Atemtrakt gehören Nase, Rachen, Kehlkopf, Luftröhre, Bronchien, Bronchiolen und die Lungenbläschen, auch Alveolen genannt. Die Luftröhre gabelt sich kurz vor dem Eintritt in die Lunge in den linken und rechten Luftröhrenhauptast, auch Stammbronchus oder Bronchus principalis genannt.

Die Lunge ist ein Teil des Atemtraktes und besteht aus Luftführenden Kanälen, dem Bronchialsystem, das die Umgebungsluft bis in die Alveolen führen, in denen Gas mit dem Blut ausgetauscht wird, also der Umgebungsluft Sauerstoff entnommen wird und Kohlendioxyd zugefügt wird. Die Alveolen bestehen aus sehr zahlreichen und sehr kleinen Bläschen, deren Durchmesser kleiner als ein Millimeter ist. Bei einem erwachsenen Menschen wird die innere Fläche aller Alveolen auf eine Größe von rund 100 Quadratmetern geschätzt.

Die Hohlräume des Atemtraktes, die die Luft vom Mund bis zu den Alveolen führen, werden als "anatomischer Totraum" bezeichnet, da sie nicht am Gasaustausch beteiligt sind. Stattdessen dienen sie zur Reinigung, Vorwärmung und Anfeuchtung der Atemluft.

Da die Lunge leider öfters mit Krankheiten zu kämpfen hat und/oder krankhaft verändert sein kann, jedoch eine direkte Diagnose der Lunge selbst mangels Sichtbarkeit nicht möglich ist, werden bildgebende Verfahren wie Röntgengeräte, Magnetresonanztomographen (MRT) und Ultraschallgeräte benutzt, um prinzipielle Aussagen über die Veränderung der Lungen treffen zu können. Diese Verfahren ermöglichen jedoch nur qualitative Diagnosen über Veränderungen der Lungen. Ein weiteres Problem ist, dass die Atmung des Menschen während der Diagnose nicht unterbrochen werden darf und Idealerweise nicht einmal behindert werden sollte.

Deshalb sind auf aktuellem Stand der Technik die verschiedensten Messgeräte zur quantitativen Erfassung der Lungenfunktion als Diagnosehilfe für zahlreiche Lungenkrankheiten bekannt, die mit mehr oder weniger hoher Genauigkeit den Flow der Atemluft, also die Luftmasse, und die Atemluftdichte beim Einatmen und beim Ausatmen messen.

Einige bekannte Messgeräte verlangen, dass der Patient voll ausatmet, voll einatmet und/oder kurze Zeit gar nicht atmet. Die Praxis zeigt, dass diese Atemkommandos nicht nur als unangenehm und unkomfortabel empfunden werden, sondern sehr häufig auch fehlerhaft, stark verzerrt oder verzögert befolgt werden, wodurch große Messfehler entstehen können, die den diagnostischen Nutzen dieser Geräte stark einschränken.

Ein weiterer Nachteil vieler bekannter Verfahren ist, dass die Konzentration des $CO_2$-Anteils der Luft über aufwändige Massenspektrometer oder relativ langsame IR-Analysatoren erfasst wird. Diese Geräte sind zumeist sehr komplex in der Herstellung und in der Bedienung und erfordern in der Regel eine Kooperation des Patienten. Deshalb sind sie für Kleinkinder, multimorbide und/oder schwer kranke sowie demente Patienten nicht einsetzbar. Ein weiterer Nachteil sind die recht hohen Kosten und das große Bauvolumen der Gesamtanordnung einschließlich aller Peripheriegeräte.

Allen Geräten gemeinsam ist, dass sie nicht in der Lage sind, direkt zwischen der sogenannten "Totraumluft" aus dem Totraum des Lungensystems und der so genannten "Alveolarluft" aus den aktiv an dem Gasaustausch beteiligten Lungenbläschen- den Alveolen - unterscheiden zu können. Der anatomische Totraum besteht aus dem Mund- und Rachenraum, aus der daran anschließenden Luftröhre, der Trachea und innerhalb der Lunge aus den Bronchien, die sich von der Luftröhre ausgehend in die beiden Lungen hinein erstrecken und dort immer weiter verästeln, bis sie die Lungenbläschen erreichen.

Nach einhelliger Lehrmeinung nehmen die Toträume überhaupt nicht oder nur in marginalem Umfang am Gasaustausch von der Atemluft in die Blutbahn teil. Die Funktion der Toträume ist die Reinigung, die thermische Konditionierung (Erwärmung) der Atemluft und die Sättigung der eingeatmeten Luft auf 100 % relative Luftfeuchtigkeit. Daher kann auch für eine genaue Ermittlung der tatsächlich in die Alveolen gelangten Luft, der Alveolarluft, das Totraumvolumen vom gesamten Atemvolumen abgezogen werden.

Auf dem bisherigen Stand der Technik ist der Beginn des Ausatmens mit guter Genauigkeit erfassbar. Jedoch nicht mit der wünschenswerten Genauigkeit erfassbar ist der Übergang von der beim Ausatmen ausgestoßenen Totraumluft zur Alveolarluft, also der aus den Lungenbläschen wieder zurück kommenden Luftmenge. Alle bisher bekannten Geräte und beschriebenen Prinzipien sind zu langsam, zu ungenau oder beides.

Die Offenlegungsschrift DE 1 918 566, Erich Jäger, vom 11.04.1969, beschreibt ein "Gerät zur Untersuchung der Lungenfunktion", bei dem sich vor der eigentlichen Messung ein "Magnetventil" öffnet, sodass der "dem Totraum entsprechende Teil der Exspirationsluft" durch den Auslass abströmt. Es wird also ganz pauschal ein bestimmter Anteil der ausgeatmeten Luft zuerst einmal als Totraumluft eingestuft.

Dieses Gerät verzichtet also von vornherein auf eine genaue Erfassung des Totraumvolumens, sondern beschränkt sich darauf, einen derart großen Anteil der ausgeatmeten Luft ungemessen ins Freie auszustoßen, dass die Messung nur mit Alveolarluft erfolgt. Dieses Prinzip bedingt jedoch, dass nur bestimmte Verhältnisse und Zusammensetzungen in der Luft gemessen werden können, nicht aber deren Volumen.

Ein krankhaft bedingt, verzerrtes Verhältnis zwischen Totraum und Alveolarraum kann also mit einem solchen Messgerät nur indirekt und daher nur mit sehr beschränkter Genauigkeit erfasst werden.

Die Offenlegungsschrift DE 28 12 379, Udo Smidt, beschreibt ein Gerät zur Lungendiagnostik, insbesondere zur Diagnostik des Lungenemphysems, das als Indikator den gegenüber gesunden Personen bei der Exspiration stark erhöhten Anteil des Mischluftvolumens wertet. Dazu wird bevorzugt ein Gasanalysator eingesetzt, dessen $CO_2$-Messfühler nach dem Infrarot-Absorptions-Prinzip arbeitet. Zusätzlich ist ein Atemstromrezeptor erforderlich, um das Atemvolumen zu bestimmen. Aus dem Verlauf der $CO_2$-Konzentration über der Zeit und dem Atemvolumen über der Zeit wird dann der Bereich konstanter Steigung der $CO_2$-Konzentration nach dem Ausatmen der Totraumluft zur Berechnung des Mischluftvolumens genutzt. Der Anstieg des Mischluftvolumens im Verhältnis zum Gesamtvolumen wird als Indikator für den Schweregrad des Lungenemphysems ausgewertet. Das Verfahren konnte sich zum Zeitpunkt seiner Offenlegung wegen des damals zu hohen Aufwandes bei der Durchführung der Rechenfunktionen, also mangels der heute nach aktuellem Stand der Technik verfügbaren Mikroprozessoren, nicht durchsetzen. Ein weiterer Nachteil ist der damals bevorzugte aufwändige Infrarotabsorptionsmessfühler.

Mit moderner Mikroprozessortechnik und besseren Sensoren könnte dieses Verfahren auf aktuellem Stand der Technik realisiert werden. Auch dann bleibt als Nachteil, dass die Erfassung des tatsächlichen, also des funktionellen Totraumvolumens nicht möglich ist, sondern nur indirekt mit einer sehr hohen Toleranz abschätzbar ist.

Mangels meist zu langsamer Messverfahren ist es kaum bekannt, dass der Übergang von der Totraumluft zur Alveolarluft durch den dramatischen Abfall der Kurve der Atemluftdichte über der Zeit auf einen für eine kurze Zeit konstanten Wert markiert wird. Vielleicht ist das eine Begründung dafür, dass bislang kein Messverfahren geschildert worden ist, das es erlaubt, diesen Punkt mit der wünschenswerten Genauigkeit zu bestimmen.

So zeigt z. B. die in der DE 28 12 379 unter Figur 2 veröffentlichte Kurve der $CO_2$-Konzentration über der Zeit diesen Abfall überhaupt nicht.

Der Anteil am Exspirationsvolumen lässt sich mit der Bohrschen Totraumformel errechnen. Mit den folgenden Abkürzungen

Vat = anatomisches Totraumvolumen
Vg = Atemzugvolumen
Di = $CO_2$-Konzentration Frischluft
De = $CO_2$-Konzentration Exspirat
Da = $CO_2$-Konzentration Alveolarluft

gelten folgende Abläufe und Beziehungen:

Vor der Inspiration sind Alveolar- und Totraum noch mit Alveolargas gefüllt. Nach der Inspiration des Atemzugvolumens Vg hat sich der Alveolarraum um Vat erweitert, jedoch nur der Anteil Vg - Vat erreicht den Alveolarraum, während der Rest im Totraum bleibt. Der Anteil im Alveolarraum vermischt sich mit dem Alveolargas, so dass dieses aufgefrischt wird. Bei der Exspiration wird zunächst Frischluft aus dem Totraum ausgeatmet, danach Alveolargas. Die Gaskonzentration erreicht danach wieder den gleichen Wert wie vor der Inspiration. Für die Beziehung der einzelnen Größen zueinander gilt die Bohrsche Formel:

$$Vat \cdot Di + (Vg - Vat) \cdot Da = Vg \cdot De$$

$$Vat = Vg \cdot \frac{Da - De}{Da}$$

Eine Diagnose nach diesem Prinzip erfordert zuerst einmal die korrekte Zuordnung zu den Bereichen Frischluft (Di), Exspirat (De) und der Alveolarluft (Da) und innerhalb dieses Bereiches dann jeweils eine korrekte Messungen der $CO_2$-Konzentrationen. Nach der Bohrschen Formel kann dann der Totraum errechnet werden. Das Ergebnis ist jedoch nur so genau, wie die Proportionalität der jeweiligen $CO_2$-Konzentrationen erhalten bleibt, was sich jedoch insbesondere bei einer erkrankten Lunge durch andere Effekte verändern kann. Deshalb ist auf der Basis der Bohrschen Formel nur eine tendenzielle, aber keine genaue Diagnose möglich.

Das Dokument US-A-2001/0049478 offenbart ein Verfahren zur Bestimmung des anatomischen Totraumes im Atemtrakt von Lebewesen durch die kontinuierliche Messung des Flow und der $CO_2$-Konzentration der Atemluft beim Ausatmen über die Zeit hinweg. Der Beginn des Ausatmens und der Totraumendpunkt werden bestimmt, und der Flow wird zwischen diesen beiden Zeitpunkten integriert. Der Totraumendpunkt wird als der Zeitpunkt bestimmt, an dem sich das Krümmungsverhalten der $CO_2$-Konzentration ändert (Wendepunkt). Auf diesem Hintergrund hat sich die Erfindung die Aufgabe gestellt, ein Verfahren zu einer genaueren Messung des anatomischen Totraumvolumens über die genaue Erfassung des Wechsels von der austretenden Totraumluft zur Alveolarluft zu entwickeln.

Als Lösung präsentiert die Erfindung, dass die Zeit vom Beginn des Ausatmens bei dem der Flow größer als Null wird bis zu einem als Totraumendpunkt bezeichneten Zeitpunkt, an dem die Atemluftdichte nach dem auf den Beginn folgenden, deutlichen Abfallen auf einen etwa konstanten Wert übergeht, gemessen wird und das Integral des Flow vom Beginn bis zum Totraumendpunkt gebildet wird, wobei die Messungen der Atemluftdichte und des Flows vom Beginn bis zum Totraumendpunkt und während der Kurzzeit als jener Zeitspanne, in welcher die Atemluftdichte den etwa konstanten Wert einnimmt, jeweils mehrfach erfolgen.

Das Integral des Flow vom Beginn des Ausatmens bis zum Totraumendpunkt entspricht mit höherer Genauigkeit als bei allen bisherigen Verfahren dem anatomischen Totraumvolumen des Atemtraktes, in dem kein Gasaustausch zwischen der Atemluft und dem Blut stattfindet. Als "Totraumendpunkt" wird der Zeitpunkt bezeichnet, an dem sämtliche im anatomischen Totraum befindliche Atemluft diesen verlassen hat.

Dieser Zeitpunkt ist dadurch charakterisiert, dass die Atemluftdichte nach dem Beginn des Ausatmens und dem daran anschließenden Abfallen auf einen etwa konstanten Wert übergeht. Die Genauigkeit, mit der dieser Zeitpunkt bestimmt wird, bestimmt auch die Genauigkeit der Ermittlung des anatomischen Totraums und ist damit ein entscheidendes Merkmal der Erfindung.

Dabei ist die größte Herausforderung der relativ schnelle Ablauf, denn das Abfallen der Atemluftdichte erstreckt sich z.B. für einen erwachsenen Menschen über einen Zeitraum von nur etwa rund 100 Millisekunden und schwankt dann für weitere 100 Millisekunden um einen etwa konstanten Wert.

Es leuchtet ohne weiteres ein, dass eine genaue Messung dieses Kurvenverlaufes mehrere Messpunkte erfordert. Deshalb schlägt die Erfindung vor, dass die Atemluftdichte und der Flow vom Beginn des Ausatmens bis zum Totraumendpunkt wenigstens etwa 100-mal gemessen werden sollte. Zu bevorzugen ist, dass die Messung wenigstens 500-mal erfolgt.

Da der dann erreichte, etwa konstante Wert auch schwankt, muss auch während dieser Zeit mehrfach gemessen werden. Vorgeschlagen wird, dass die Atemluftdichte und der Flow in dieser Zeit eines etwa konstanten Wertes-wenigstens etwa 30-mal, vorzugsweise jedoch wenigstens etwa 150-mal gemessen werden.

Zur Auswertung des Kurvenverlaufs sollte in einer Ausführungsvariante in einer übergeordneten Steuerung aus den beiden Bereichen des Abfallens und eines etwa konstanten Wertes jeweils ein Mittelwert gebildet werden, sodass im Ergebnis zwei sich kreuzende Graden auf der Zeitachse mit relativ hoher Genauigkeit den Totraumendpunkt markieren.

Die Erfindung schlägt als Verfahren zur Ermittlung dieser beiden Graden vor, dass in einem Speicher der Verlauf der Atemluftdichte hinterlegt wird. Aus den gespeicherten Werten kann dann mit den folgenden drei Schritten der Totraumendpunkt recht genau ermittelt werden:

Im ersten Schritt wird aus dem Verlauf der Atemluftdichte der niedrigste Wert und eine vorwählbare Anzahl der nächst niedrigen Werte selektiert und in dem davon überstreckten Zeitbereich der Mittelwert aus allen Messungen gebildet und als konstanter Wert gewertet.

Im zweiten Schritt wird aus dem Bereich vom Beginn des Ausatmens bis zum erstmaligen Erreichen des konstanten Wertes ein Mittelstück der Kurve mit einer vorwählbaren Breite selektiert und aus allen Messwerten dieses Bereiches eine Grade mit einer bestimmten Steigung ermittelt.

Im dritten Schritt wird der Kreuzungspunkt dieser Geraden mit dem ermittelten Wert für den konstanten Wert als Zeitpunkt für den Totraumendpunkt gewertet.

Für die Durchführung solcher-Rechenoperationen sind auf aktuellem Stand der Technik Mikroprozessoren und dazu passende Software bekannt, die mit vollauf ausreichender Geschwindigkeit und ausreichender Auflösung arbeiten.

Da bei der Messung auch stets das Messraumvolumen der Messstrecke mit erfasst wird, muss dieses Volumen vom Messergebnis subtrahiert werden. Da das Innenvolumen der Messstrecke sehr genau bekannt ist und da die Strömung innerhalb der Messstrecke auch nahezu laminar verläuft, kann das Messraumvolumen direkt vom Messergebnis subtrahiert werden.

Wenn z. B. die Messsensoren in der Mitte der Messstrecke angeordnet sind, dann ergibt sich der anatomische Totraum als die Summe des gemessenen Volumens vom Beginn des Ausatmens bis zum Totraumendpunkt vermindert um die Hälfte des Messraumvolumens. oder als Formel

$$Vat = \sum_{To}^{Tt} Vg - \tfrac{1}{2}\, Vap$$

Dabei ist

Vat      das anatomische Totraumvolumen,
Vg       das in der Messstrecke gemessene Atemvolumen,
Vap     das Messraumvolumen der Messstrecke,
To       der Beginn des Ausatmens und
Tt        der Totraumendpunkt, der das Ende der Zeit vom Beginn To des Ausatmens bis zum vollständigen Ausstoß der in den anatomischen Totraum (Vat) eingeatmeten Luft markiert.

Das erfindungsgemäße Verfahren zur Lungentotraummessung kann für jedes Lungendiagnosegerät angewandt werden, dass eine Messstrecke zur Messung des Flows und der Atemluftdichte der Atemluft in Abhängigkeit von der Zeit aufweist und mit einem Speicher zur Speicherung der Messwerte und einem Rechner, wie z. B. einem Mikroprozessor, zur Unterstützung der Messung und zur Auswertung der gespeicherten Messungen ausgerüstet ist. Dieses Gerät muss auch über eine Anzeige oder zumindest eine Ausgabeeinheit für die Messergebnisse verfügen.

Ein wesentliches Merkmal der Erfindung ist, dass alle Messungen so schnell wiederholt werden, dass die Werte mit einer ausreichenden Genauigkeit erfasst werden. Als allgemeine Faustregel gilt eine Wiederhol-Frequenz von wenigstens 1 Kilohertz wiederholt. Bei niedrigeren Frequenzen sind entsprechende Abstriche in der Genauigkeit zu machen.

Eine sehr interessante Ausführungsform der Messstrecke ist eine Ultraschallmessstrecke, die unter ei-

nem Winkel zur Atemrichtung geneigt ist. Mit dieser Messstrecke ist die Laufzeit eines schräg zum Atemstrom gerichteten Ultraschallimpulses während eines Atemzuges sehr häufig messbar, sodass daraus in einer Auswerteeinheit die aktuellen Werte des Flows (Luftmasse) und der Atemluftdichteerrechnet werden können. Diese Werte sind die Grundlage für die Durchführung des erfindungsgemäßen Verfahrens.

Ein Lungendiagnosegerät, in dem das erfindungsgemäße Verfahren implementiert ist, kann mit zusätzlichen, diagnostischen Funktionen ausgestattet werden. So ist es z. B. denkbar, dass ermittelte Verhältnis zwischen Alveolarluft und Totraumvolumen mit einem Normalwert zu vergleichen und als Ergebnis einen Verhältniswert zu erzeugen.

Dieser Verhältniswert könnte direkt am Gerät angezeigt werden, z.B. durch Ziffern, durch eine Analoganzeige und/oder durch eine Quasi-Analog-Anzeige. Eine andere Variante sind drei, jeweils einstufige Anzeigeelemente, wie z. B. Leuchtdioden, die entweder leuchten oder dunkel sind. Eine sehr einfache und deshalb auch durch den Patienten selbst zu interpretierende Anzeigereihenfolge wäre eine grüne Leuchtdiode, wenn das Verhältnis im normalen Bereich liegt; eine gelbe Leuchtdiode, wenn das Verhältnis gegenüber dem Normalwert etwas abweicht und eine rote Leuchtdiode, wenn das Verhältnis in kritischem Umfang vom Normalbereich abweicht.

Alle mit einer Ultraschallmesstrecke nach dem erfindungsgemäßen Verfahren arbeitenden Lungendiagnosegeräte haben den Vorteil, dass sie auf eine genaue Ermittlung des absoluten Wertes der Atemluftdichte verzichten können und stattdessen nur ein Verhältnis der $CO_2$-Dichten erfassen müssen, wofür eine sehr hohe, reproduzierbare Genauigkeit erreichbar ist.

Dadurch kann das aufwändige Massenspektrometer entfallen und es wird möglich, eine sehr kleine und kompakte Einheit zu produzieren, die sogar als ein in der Hand haltbares Kompaktgerät ausführen kann.

Ein weiterer Vorteil ist, dass keinerlei Atemkommandos mehr gegeben werden müssen, weshalb eine solche Einheit auch zur Lungendiagnose von Kindern, Kleinkindern und Babys geeignet ist, wie z. B. zur Beurteilung des normalen Wachstums der Lunge.

Ein anderes, wesentlichen Anwendungsgebiet ist die Veterinärmedizin, bei der jede Art von Atemkommandos undenkbar sind und schon das Anlegen der Messstrecke vor dem Atemweg des Tieres eine Herausforderung ist. Hier ermöglicht das erfindungsgemäße Verfahren erstmals überhaupt eine Lungendiagnostik für zahlreiche, verschiedene Tiere und ihre diversen Lungenkrankheiten.

Das erfindungsgemäße Verfahren vereinfacht also die qualitative und quantitative Diagnose von Lungenemphysemen deutlich. Dieses Krankheitsbild bezeichnet die irreversible Überblähung der belüfteten Räume der terminalen Bronchiolen, also der kleinsten, luftgefüllten Lungenstrukturen am Ende des Totraums der Lunge vor dem Übergang in den Alveolarraum, also vor dem Übergang zu den gasaustauschenden Lungenbläschen.

Durch den Verlust der Elastizität des Lungengewebes kann die enthaltene Luft nicht mehr vollständig entweichen, wodurch sich der Druck auf die Alveolen erhöht, diese kollabieren lässt und dadurch Lufträume im Alveolarbereich einschließt, die nicht mehr abgeatmet werden können. Dadurch werden im Extremfall aus zuvor noch funktionstüchtigen Lungenbläschen große funktionslose "Emphysemblasen".

Dieses Krankheitsbild kann mit hilfe des erfindungsgemäßen Verfahrens zur Lungentotraummessung nicht nur auf reines Auftreten beurteilt werden, sondern zusätzlich auch quantifiziert werden, also einem bestimmten Schweregrad der Erkrankung zugeordnet werden.

Im Folgenden sollen weitere Einzelheiten und Merkmale der Erfindung anhand eines Beispiels näher erläutert werden. Dieses soll die Erfindung jedoch nicht einschränken, sondern nur erläutern. Es zeigt in schematischer Darstellung:

Figur 1    Symbolisierter Schnitt durch einen menschlichen Torso mit Lungendiagnosegerät

Figur 2    Prinzipielle Verläufe der Atemluftdichte und des Flows

Die Figuren zeigen im Einzelnen:

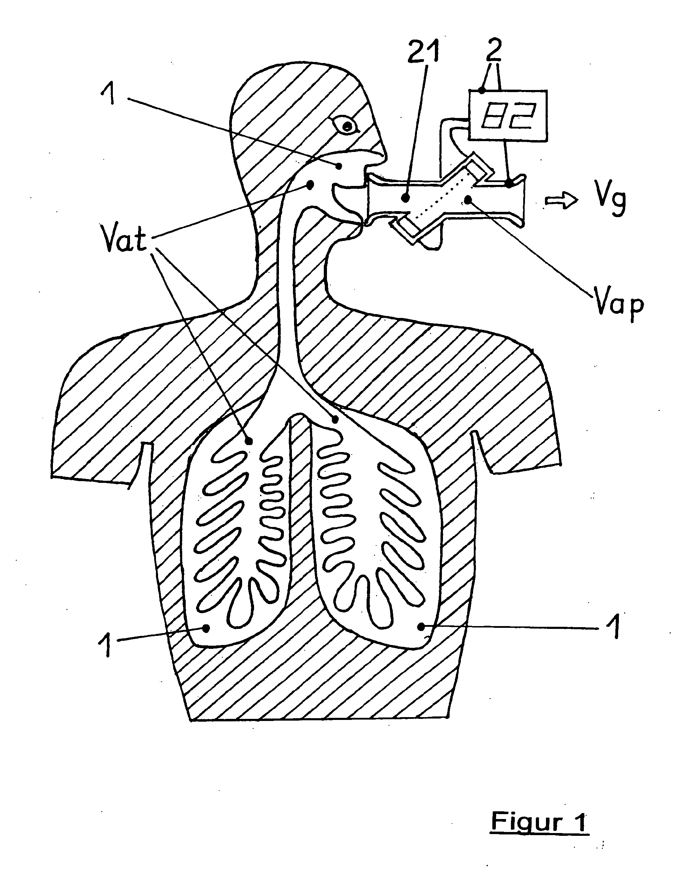

In **Figur 1** ist stark vereinfacht und stilisiert der Querschnitt durch einen menschlichen Torso gezeichnet, in dem der Atemtrakt (1) symbolhaft eingetragen ist. Der Teil des Atemtraktes vom Mundraum bis in die Bronchien ist das anatomische Totraumvolumen (Vat). Daran anschließend, innerhalb der Lunge werden die Bronchien von den Alveolen umgeben, die in Figur 1 nicht einzeln eingezeichnet sind, sondern durch die Fläche zwischen den symbolhaft skizzierten Bronchien im Inneren und dem äußeren Umriss der Lunge wiedergegeben werden. Dadurch wird in Figur 1 nachvollziehbar, dass sich der gesamte Luftführungsraum im menschlichen Körper aufteilt in den Totraum (Vat) und den Alveolarraum.

Figur 1 zeigt, wie dem Patienten ein Lungendiagnosegerät (2) an den Mund angelegt wird. Es enthält eine Messstrecke (21), die sich vom Mundstück bis zur Öffnung für das Ausatmen erstreckt. In deren Mitte sind zwei Ultraschallsender und -empfänger so angeordnet, dass der Schall in einem Winkel zur Atemrichtung verläuft, was durch eine gepunktete Linie symbolisiert wird. Auf diese Weise wird die Laufzeit des Ultraschalls und dessen Beeinflussung durch die Atemluft messbar.

In einer - hier nicht dargestellten - Auswerteeinheit werden die Messwerte gespeichert und ausgewertet und können dann - z.B. wie in Figur 1 gezeichnet - durch eine einfache Siebensegmentanzeige am oberen Teil des Lungendiagnosegerätes (2) ausgegeben werden.

In Figur 1 wird das gesamte, in der Messstrecke gemessene Volumen (Vg) durch einen Doppelpfeil vor der Messstrecke 21 dargestellt. Es ist sehr gut nachvollziehbar, dass das gesamte gemessene Volumen (Vg) die Summe aus dem anatomischen Totraumvolumen (Vat) und dem - hier nicht näher bezeichneten - Alveolarvolumen sowie dem halben Messraumvolumen (Vap) der Messstrecke ist.

In Figur 1 ist ersichtlich, dass die Ultraschallstrecke die Messstrecke 21 in ihrer Mitte kreuzt. Daher wird die - in Figur 1 links gezeichnete - Hälfte der Messstrecke mit in der Messung erfasst.

In Figur 1 wird ebenfalls sehr schnell deutlich, dass der Patient zur Benutzung des Gerätes nahezu unbehindert durch die hier rohrförmige Messstrecke 21 atmen kann.

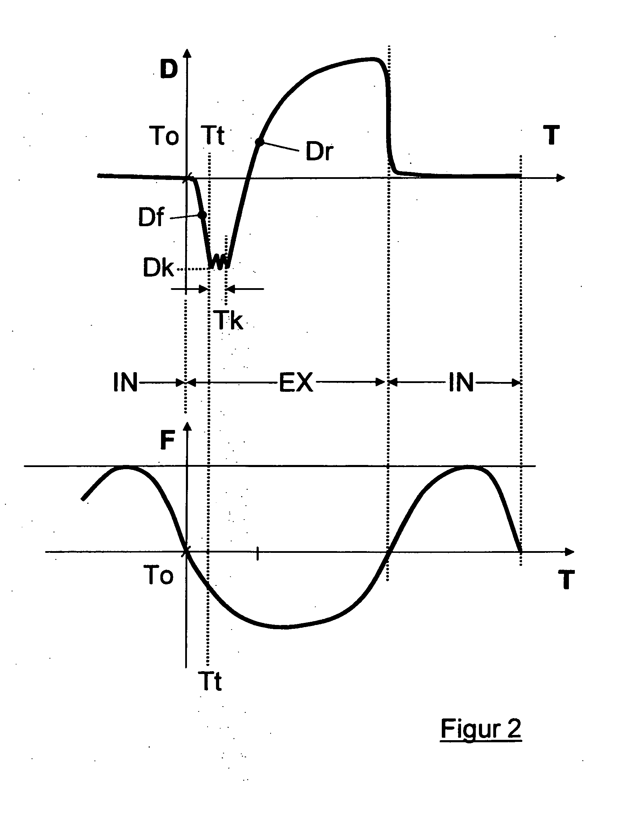

In **Figur 2** ist in der oberen Kurve der prinzipielle Verlauf der Atemluftdichte über einen Zyklus, bestehend aus dem Ausatmen (EX) und dem Einatmen (IN) gezeichnet. Im unteren Teil ist auf der gleichen Zeitachse der Verlauf des Flows (F) eingetragen.

In der oberen Kurve, der Atemluftdichte(D), ist deutlich zu erkennen, dass mit dem Beginn des Ausatmens (EX) die Atemluftdichte mit der fallenden Flanke (Df) steil abfällt, bis sie den konstanten Wert (Dk) erreicht. Für die Kurzzeit (Tk) schwankt die Atemluftdichte(D) etwas um den konstanten Werk (Dk) und geht dann in die ansteigende Flanke (Dr) über. Mit dem Ende des Ausatmens (EX) und dem Beginn des Einatmens (IN) fällt die Atemluftdichte(D) schlagartig wieder auf Null.

In der oberen Kurve ist in Figur 2 sehr gut nachvollziehbar, dass die fallende Flanke (Df) mit begrenztem, rechnerischem Aufwand durch eine Gerade angenähert werden kann.

Ebenso zeigt Figur 2, dass die Schwankungen um den konstanten Wert (Dk) mit hoher Genauigkeit durch einen einzigen Mittelwert (Dk) zusammengefasst werden können. Wenn dieser Mittelwert (Dk) als eine - zur Zeitachse parallele - Gerade aufgetragen wird, dann wird in Figur 2 sehr schnell plausibel, dass der Kreuzungspunkt dieser Geraden mit der ebenfalls durch eine Gerade ersetzten, fallenden Flanke (Df) des Verlaufs der Atemluftdichte mit relativ hoher Genauigkeit den Totraumendpunkt (Tt) wiedergibt.

Die untere Kurve, der Flow F, über der Zeit (T) macht nachvollziehbar, dass zusammen mit dem - bekannten - Volumen der Messstrecke (21) das Totraumvolumen (Vat) exakt berechnet werden kann.

## Bezugszeichenliste

| | |
|---|---|
| 1 | Lungensystem |
| 2 | Lungendiagnosegerät |
| 21 | Messstrecke des Lungendiagnosegerätes (2) |
| D | Atemluftdichte beim Ausatmen (EX) |
| Df | fallende Atemluftdichte nach dem Beginn des Ausatmens (EX) |
| Dk | konstanter Wert der Atemluftdichte |
| Dr | ansteigende Flanke des Verlaufs der Atemluftdichte |
| EX | Ausatmen, engl. Exhale |
| F | Flow, Geschwindigkeit der Atemluft beim Einatmen (IN) und beim Ausatmen (EX) |
| IN | Einatmen, engl. Inhale |
| T | Zeit |
| To | Beginn des Ausatmens (EX) |
| Tk | Kurzzeit, während der die Atemluftdichte etwa auf dem Wert Dk verharrt |
| Tt | Totraumendpunkt, Zeit vom Beginn To des Ausatmens bis zum vollständigen Ausstoß der in den anatomischen Totraum (Vat) eingeatmeten Luft |
| Vap | Messraumvolumen der Messstrecke 21 |
| Vat | anatomisches Totraumvolumen |
| Vg | in der Messstrecke 21 gemessenes Volumen |

## Patentansprüche

1. Verfahren zur Bestimmung des anatomischen Totraumes (Vat) im Atemtrakt (1) von Lebewesen durch die kontinuierliche und simultane Messung

   - des Flow (F) und
   - der Atemluftdichte(D) der Atemluft beim Ausatmen (EX) über die Zeit (T) hinweg, wobei
   - die Zeit
   - vom Beginn (To) des Ausatmens (EX) bei dem der Flow (F) größer als Null wird
   - bis zu einem als Totraumendpunkt (Tt) bezeichneten Zeitpunkt, an dem die Atemluftdichte (D) nach dem auf den Beginn (To) folgenden, deutlichen Abfallen (Df) auf einen etwa konstanten Wert (Dk) übergeht, gemessen wird und
   - das Integral des Flow (F) vom Beginn (To) bis zum Totraumendpunkt (Tt) gebildet wird, wobei
   - die Messungen der Atemluftdichte(D) und des Flows (F)
   - vom Beginn (To) bis zum Totraumendpunkt (Tt) und
   - während der Kurzzeit (Tk) als jener Zeitspanne, in welcher die Atemluftdichte(D) den etwa konstanten Wert (Dk) einnimmt, jeweils mehrfach erfolgen.

2. Verfahren nach dem vorhergehenden Anspruch 1, **dadurch gekennzeichnet, dass** die Messungen

   - der Atemluftdichte(D) und
   - des Flows (F)
   vom Beginn (To) bis zum Totraumendpunkt (Tt) wenigstens etwa einhundert Mal erfolgen.

**3.** Verfahren nach dem vorhergehenden Anspruch 2, **dadurch gekennzeichnet, dass** die Messungen wenigstens etwa fünfhundert Mal erfolgen.

**4.** Verfahren nach einem der vorhergehenden Ansprüche, **dadurch gekennzeichnet, dass** die Messungen

- der Atemluftdichte(D) und
- des Flows (F)

während der Kurzzeit (Tk) wenigstens etwa dreißig Mal erfolgen.

**5.** Verfahren nach dem vorhergehenden Anspruch 4, **dadurch gekennzeichnet, dass** die Messungen wenigstens etwa einhundertfünfzig Mal erfolgen.

**6.** Verfahren nach einem der vorhergehenden Ansprüche, **dadurch gekennzeichnet, dass** zur genauen Ermittlung des Totraumendpunktes (Tt), ab dem die Atemluftdichte(D) um den konstanten Wert (Dk) schwankt, der Verlauf der Atemluftdichte(D) gespeichert wird und

- im ersten Schritt daraus der niedrigste Wert und eine vorwählbare Anzahl der nächst niedrigen Werte selektiert werden und in dem davon überstreckten Zeitbereich der Mittelwert aus allen Messungen gebildet und als konstanter Wert (Dk) gewertet wird und
- im zweiten Schritt aus dem Bereich vom Beginn des Ausatmens bis zum erstmaligen Erreichen des konstanten Wertes (Dk) ein Mittelstück der Kurve mit einer vorwählbaren Breite selektiert wird und aus allen Messwerten dieses Bereiches eine Gerade mit einer bestimmten Steigung ermittelt wird und
- im dritten Schritt der Kreuzungspunkt dieser Graden mit dem ermittelten Wert für den konstanten Wert (Dk) als Zeitpunkt für den Totraumendpunkt (Tt) gewertet wird.

**7.** Lungendiagnosegerät (2), in dem das Verfahren nach einem der vorhergehenden Ansprüch implementiert ist, wobei das Lungendiagnosegerät (2)

- eine Messstrecke (21) zur Messung des Flows (F) und der Atemluftdichte(D) der Atemluft über die Zeit (T) und
- einen Speicher zur Speicherung der Messwerte und
- einen Rechner, wie z.B. einen Mikroprozessor, zur Unterstützung der Messung und zur Auswertung der gespeicherten Messungen und
- eine Anzeige- oder Ausgabeeinheit für die Messergebnisse aufweist.

**8.** Lungendiagnosegerät (2) nach dem vorhergehenden Anspruch 7, **dadurch gekennzeichnet, dass** die Messstrecke (21)

- auf den Lufteintritt des Atemtraktes (1) aufgesetzt ist und
- ein Messraumvolumen (Vap) aufweist und
- in ihrer Mitte einen Messsensor aufweist und das anatomische Totraumvolumen (Vat) die Summe des gemessenen Volumens (Vg) vom Beginn (To) des Ausatmens (EX) bis zum Totraumendpunkt (Tt), vermindert um die Hälfte des Messraumvolumens (Vap) ist, entsprechend der Formel

$$Vat = \sum_{To}^{Tt} Vg - \tfrac{1}{2}\,Vap\;.$$

**9.** Lungendiagnosegerät (2) nach dem vorhergehenden Anspruch 8, **dadurch gekennzeichnet, dass** die Messstrecke (21) eine Ultraschallmessstrecke ist, die unter einem Winkel zur Atemrichtung geneigt ist.

**Claims**

**1.** Method for determining the anatomical dead space (Vat) in the respiratory tract (1) of living organisms by the continuous and simultaneous measurement

- of flow (F) and
- respiratory air density (D)

of the respiratory air during exhalation (EX) over the time (T), wherein

- the time
- from the start (To) of exhalation (EX) at which the flow (F) becomes greater than zero
- until a time designated the dead space end point (Tt), at which the respiratory air density (D), after the significant decreases (Df) following the start ($T_0$), becomes an approximately constant value (Dk),

is measured, and

- the integral of the flow (F) from the start ($T_0$) until the dead-space end point (Tt) is formed, wherein
- the measurements of respiratory air density (D) and flow (F)
- From the start (To) until the dead space end point (Tt) and

- during the short time (Tk) as that time span in which the respiratory air density (D) assumes the approximately constant value (Dk),

are made repeatedly in each case.

2. Method according to the preceding claim 1, **characterised in that** the measurements

- of the respiratory air density (D) and
- of the flow (F)

from the start ($T_0$) until the dead space end point (Tt) are made at least one hundred times.

3. Method according to the preceding claim 2, **characterised in that** the measurements are made approximately five hundred times.

4. Method according to one of the preceding claims, **characterised in that** the measurements

- of the respiratory air density (D) and
- flow (F) during the short time (Tk)

are made at least approximately thirty times.

5. Method according to the preceding claim 4, **characterised in that** the measurements are made at least approximately one hundred and fifty times.

6. Method according to one of the preceding claims, **characterised in that** for precise determination of the dead space end point (Tt), from which the respiratory air density (D) fluctuates about the constant value (Dk), the profile of the respiratory air density (D) is stored, and

- in the first step the lowest value and a preselectable number of the next low values are selected, and, in the time range covered thereby, the average value of all the measurements is formed and evaluated as a constant value (Dk), and
- in the second step, from the range from the start of exhalation until the constant value (Dk) is reached for the first time, a central portion of the curve with a preselectable width is selected, and, from all the measurement values of this range, a straight line with a particular gradient is determined, and
- in the third step, the intersection of these straight lines with the value determined for the constant value (Dk) is evaluated as the time point for the dead-space end point (Tt).

7. Lung diagnosis apparatus (2), in which the method according to one of the preceding claims is implemented, wherein said lung diagnosis apparatus (2) comprises

- a measurement zone (21) for measuring the flow (F) and the respiratory air density (D) of the respiratory air over time (T), and
- a memory for storing the measurement values, and
- a computer, such as a microprocessor, for supporting the measurement and for evaluating the stored measurements, and
- a display or output unit for the measurement results.

8. Lung diagnosis apparatus (2) according to the preceding claim 7, **characterised in that** the measurement zone (21)

- is mounted on the air inlet of the respiratory tract (1), and
- comprises a measurement space volume (Vap), and
- comprises in its centre a measurement sensor, and

the anatomical dead-space volume (Vat) is the total of the measured volume (Vg) from the start ($T_o$) of exhalation (EX) until the dead space end point (Tt), reduced by half of the measurement zone volume (Vap), corresponding to the formula

$$Vat = \sum\nolimits_{T0}^{Tt} Vg - {}^{1}/_{2}\, Vap$$

9. Lung diagnosis apparatus (2) according to the preceding claim 8, **characterised in that** the measurement zone (21) is an ultrasound measurement zone, which is inclined at an angle to the breathing direction.

**Revendications**

1. Procédé destiné à déterminer l'espace mort anatomique (Vat) dans l'appareil respiratoire (1) d'êtres vivants à travers la mesure continue et simultanée

• du flux (F) et
• de la densité (D)

de l'air respiré lors de l'expiration (EX) au cours du temps, sachant que

• ce temps est mesuré
• depuis le début (To) de l'expiration (EX) pour laquelle le flux (F) est supérieur à zéro

• jusqu'au moment désigné comme point final de l'espace mort (Tt), auquel la densité de l'air respiré (D), après les chutes (Df) nettes suivant le commencement (To) passe à une valeur à peu près constante (Dk),

• l'intégralité du flux (F) étant formée depuis le début (To) jusqu'au point final de l'espace mort (Tt), sachant

• que les mesures de la densité de l'air respiré (D) et du flux (F) ont lieu à chaque fois plusieurs fois

• depuis le début (To) jusqu'au point final de l'espace mort (Tt) et

• pendant le court temps (Tk) correspondant au laps de temps au cours duquel la densité de l'air respiré (D) adopte la valeur à peu près constante (Dk).

2. Procédé d'après la revendication précédente 1, **caractérisé par le fait que** les mesures

• de la densité de l'air respiré (D) et
• du flux (F)

ont lieu au moins environ cent fois depuis le début (To) jusqu'au point final de l'espace mort (Tt).

3. Procédé selon la revendication précédente 2, **caractérisé par le fait que** les mesures ont lieu au moins cinq cent fois.

4. Procédé selon une des revendications précédentes, **caractérisé par le fait que** les mesures

• de la densité de l'air respiré (D) et
• du flux (F)

ont lieu au moins trente fois pendant le court temps (Tk).

5. Procédé selon la revendication précédente 4, **caractérisé par le fait que** les mesures ont lieu au moins environ cent cinquante fois.

6. Procédé selon une des revendications précédentes, **caractérisé par le fait que** pour déterminer exactement le point final de l'espace mort (Tt) à partir duquel la densité de l'air respiré (D) varie de la valeur constante (Dk), le tracé de la densité de l'air respiré (D) est enregistré et

• dans une première étape la valeur la plus basse qui en provient et un nombre pouvant être préalablement sélectionné des valeurs basses les plus proches sont sélectionnés, la valeur moyenne de toutes les mesures étant formée et évaluée en tant que valeur constante (Dk) dans la plage de temps parcourue,

• dans une deuxième étape, une partie médiane de la courbe provenant de la plage allant du début de l'expiration jusqu'à l'atteinte pour la première fois de la valeur constante est sélectionnée avec une largeur pouvant être préalablement sélectionnée, une ligne droite ayant une certaine pente étant déterminée à partir de toutes les valeurs de mesure de cette zone,

• dans une troisième étape, le point de croisement de ces droites étant évalué avec la valeur déterminée pour la valeur constante (Dk) en tant que moment du point final de l'espace mort (Tt).

7. Appareil de diagnostic des poumons (2) selon la revendication précédente 7, **caractérisé par le fait que** le tronçon de mesure

• est posé sur l'entrée d'air respiratoire (1), et
• présente un volume d'espace mesuré (Vap) et
• présente en son milieu un capteur de mesure, le volume de l'espace mort anatomique (Vat) étant la somme du volume mesuré (Vg) depuis le début (To) de l'expiration (EX) jusqu'au point final de l'espace mort (Tt) diminué de la moitié du volume de l'espace mesuré (Vap), conformément à la formule

$$Vat = \sum_{To}^{Tt} Vg - \frac{1}{2} Vap.$$

8. Procédé selon une des revendications précédentes, **caractérisé par le fait qu'**il est configuré en tant qu'appareil TENS ou qu'appareil EMS et

• que des consignes peuvent être données pour la pose des électrodes cutanés 63 sur un muscle donné et

• que des ordres peuvent être donnés pour la contraction maximale et la décontraction de ce muscle et

• que, pour les deux états du muscle, la résistance électrique et/ou sa modification peuvent être sauvegardées en mémoire en fonction du temps et

• peuvent être vérifiés au début de chaque période d'utilisation en tant que confirmation de l'indication correcte de l'identité de l'utilisateur respectif A, B à travers des ordres correspondants à l'utilisateur A, B.

9. Appareil de diagnostic des poumons (2) selon la revendication précédente 8, **caractérisé par le fait que** le tronçon de mesure (21) est un tronçon de mesure des ultrasons qui est incliné dans un certain angle par rapport au sens de la respiration.

Figur 1

Figur 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1918566, Erich Jäger
- DE 2812379, Udo Smidt
- US 20010049478 A